# EUROPEAN PATENT APPLICATION

(11) **EP 3 741 377 A1**
(43) Date of publication of application: **25.11.2020**
(21) Application number: 19741460.0
(22) Date of filing: 17.01.2019
(51) Int. Cl.: A61K 36/064, A23L 33/145, A61K 31/736, A61P 1/00, A61P 43/00

(54) **MUCIN GENERATION PROMOTER AND FUCOSYLATION PROMOTER, AND FOOD PRODUCT COMPOSITION CONTAINING SAME**

(30) Priority: 18.01.2018 JP 2018006230
(71) Applicant: Asahi Group Holdings, Ltd., Tokyo 130-8602 (JP)
(72) Inventor: TANIHIRO Reiko, Moriya-shi, Ibaraki 302-0106 (JP)
(74) Representative: Hoffmann Eitle
(86) International application number: PCT/JP2019/001185
(87) International publication number: WO 2019/142846

(57) **Abstract**

The present invention provides an agent having an effect of promoting production of mucin in the intestinal tract (mucin generation promoting agent) and an agent having an effect of promoting fucosylation in the intestinal tract (fucosylation promoting agent), the agents being suitable for use in food/beverage products for enhancing intestinal barrier function (food product compositions for intestinal barrier function enhancement). The agents of the present invention are a mucin generation promoting agent containing yeast mannan and a fucosylation promoting agent containing yeast mannan. The present invention also provides a food product composition for intestinal barrier function enhancement containing any of these agents.

## Description

### Technical Field

The present invention relates to an agent for promoting generation of mucin or promoting fucosylation in the intestinal tract. The present invention also relates to uses of mannan (yeast mannan) extracted from yeast cell walls.

### Background Art

Goblet cells, one of intestinal epithelial cells, secrete a large amount of the glycoprotein mucin to form mucus layers on epithelial cell surfaces, thereby preventing invasion of bacteria. Many glycans and glycosyltransferases are expressed in intestinal epithelial cells. In particular, mice with deletion of fucosyltransferase (Fut2) are susceptible to infection with Salmonella enterica, one of pathogenic bacteria. From this fact, addition of fucose (fucosylation) to the intestinal tract is inferred to play an important role in the prophylaxis system. The innate lymphocyte ILC3 produces interleukin 22 to enhance expression of the Fut2 gene, and as a result intestinal epithelial cells are fucosylated.

Patent Literature 1 (JP 2002-255848 A) discloses that α-lactalbumin, a protein contained with high concentrations in milk and dairy products, particularly in whey, has, for example, an effect of promoting mucin production/secretion from mucosal cells in the stomach or the like.

Patent Literature 2 (JP 2008-289381 A) discloses that inulin-type fructan, a polysaccharide in which a large number of fructose molecules are bonding to a molecule of sucrose, has mucin-secretion-promoting effect on the large intestine or the like and has an effect of preventing inflammatory intestinal diseases.

Bulky insoluble dietary fibers and highly viscous water-soluble dietary fibers are generally said to promote mucin secretion in the small intestine by virtue of their physical characteristics. For example, Non Patent Literature 1 reports that the amount of mucin in the small intestine increases as the bulkiness of a dietary fiber is higher for soluble dietary fibers and the viscosity of a dietary fiber is higher for water-soluble dietary fibers (e.g., glucomannan).

### Citation List

### Patent Literature

Patent Literature 1: JP 2002-255848 A
Patent Literature 2: JP 2008-289381 A

### Non Patent Literature

Non Patent Literature 1: MORITA Tatsuya, RESEARCH FOR THE MECHANISM OF MUCIN SECRETORY EFFECTS BY DIETARY FIBER AND ITS PHYSIOLOGICAL SIGNIFICANCE, Shizuoka University, May 15, 2012 (http://hdl.handle.net/10297/6999)

### Summary of Invention

### Technical Problem

Desired are foods with health claims (e.g., foods with functional claims) that can enhance a function of protecting from infection with pathogenic bacteria through secretion of mucin or fucosylation in the intestinal tract (herein referred to as "intestinal barrier function") or an agent that serves as an active ingredient of them (e.g., a functional substance). However, products having sufficient enhancing effect and practicality have not yet been developed.

Dietary fibers are known to have an effect of promoting secretion of mucin from mucosal cells, as described in Non Patent Literature 1, whereas uses of insoluble dietary fibers are restricted to particular food forms because of the insolubility, and use of water-soluble dietary fibers with high viscosity such as glucomannan for beverages is difficult because such water-soluble dietary fibers do not allow a large amount of ingestion at one time. In addition, protein components such as lactalbumin described in Patent Literature 1 undergo thermal agglomeration, which restricts processing into beverage forms.

An object of the present invention is to provide an agent having an effect of promoting generation of mucin in the intestinal tract and an agent having an effect of promoting fucosylation in the intestinal tract, in other words, a mucin generation promoting agent and a fucosylation promoting agent, the agents being suitable for use in food/beverage products for enhancing intestinal barrier function (food product compositions for intestinal barrier function enhancement).

### Solution to Problem

The present inventors found that α-mannan extracted from a yeast (herein, referred to as "yeast mannan" or "yeast α-mannan") has an effect of promoting production and secretion of mucin from the intestinal tract. Yeast mannan is water-soluble and an aqueous solution thereof has very low viscosity, and hence serves as a mucin production promoting agent suitable for use for food/beverage products.

The present inventors further found that yeast mannan has an effect of facilitating expression of fucosyltransferase (Fut2) in intestinal epithelial cells to promote addition of fucose to cell surfaces, that is, fucosylation of the intestinal tract, and accordingly yeast mannan is applicable even as a fucosylation promoting agent.

The scope of the present invention encompasses the following items.
[Item 1]
   A mucin generation promoting agent containing yeast mannan.
[Item 2]
   A fucosylation promoting agent containing yeast mannan.
[Item 3]
   A food product for intestinal barrier function enhancement composition containing the mucin generation promoting agent according to item 1 or the fucosylation promoting agent according to item 2.

### Advantageous Effects of Invention

Use of the mucin generation promoting agent or fucosylation promoting agent of the present invention, each containing yeast mannan, enables production of intestinal-barrier-function-enhancing food product compositions that are superior in intestinal-barrier-function-enhancing effect and allow easy ingestion.

### Brief Description of Drawings

[Figure 1] Figure 1 shows a graph representing a result of intergroup comparison of mucin productions (amounts of mucin in feces of mice) after a test period over 6 weeks in Experiment 1 (Promotion of Mucin Generation) in Examples. *: p < 0.05, **: p < 0.01, ***: p < 0.001.
[Figure 2] Figure 2 shows a graph representing expression levels (relative values to the expression level in Comparative Example 1 as 1.0) of the Fut2 gene after a test period over 6 weeks in Experiment 2 (Promotion of Fucosylation) in Examples. **: p < 0.01.

### Description of Embodiments

### [Mucin Generation Promoting Agent and Fucosylation Promoting Agent]

Each of the mucin generation promoting agent and fucosylation promoting agent of the present invention contain yeast mannan, and may contain only yeast mannan (consist of yeast mannan) as an active ingredient for the purpose of promotion of mucin generation or promotion of fucosylation, or contain another active ingredient for the same purpose in addition to yeast mannan.

In typical embodiments of the present invention, generation of mucin and fucosylation in the intestinal tract can be simultaneously promoted through ingestion of yeast mannan as an active ingredient of the mucin generation promoting agent and fucosylation promoting agent. Accordingly, in one embodiment of the present invention, the mucin generation promoting agent and fucosylation promoting agent can be embodied as one agent. On the other hand, the scope of the present invention also encompasses embodiments in which the operative advantageous effect of promotion of mucin generation is exerted but the operative advantageous effect of promotion of fucosylation is not exerted, and embodiments in which, inversely, the operative advantageous effect of promotion of fucosylation is exerted but the operative advantageous effect of promotion of mucin generation is not exerted.

### (1) Yeast Mannan

In the present invention, yeast mannan refers to a substance containing at least one of a yeast-derived "α-mannan" and a yeast-derived "α-mannan-protein complex". Both of the α-mannan and the α-mannan-protein complex are main components constituting yeast cell walls.

The above-mentioned "α-mannan" is a polysaccharide formed through polymerization of D-mannose via an α1,6-bond, an α1,2-bond, or an α1,3-bond, and a side chain formed of one or two or more mannopyranose moieties may be bonding to the main chain via an α1,2-bond or an α1,3-bond. This α-mannan is water-soluble.

The above-mentioned "α-mannan-protein complex" is a water-soluble glycoprotein in which one or two or more α-mannan chains are bonding to a protein locally present in yeast cell walls via an N-glycoside bond or an O-glycoside bond. As with the case with the above "α-mannan", each of the "α-mannan chains" bonding to the glycoprotein is a polysaccharide chain formed through polymerization of D-mannose via an α1,6-bond, an α1,2-bond, or an α1,3-bond, and a side chain formed of one or two or more mannopyranose moieties may be bonding to the main chain via an α1,2-bond or an α1,3-bond.

Yeast mannan may be collected in any manner as long as being derived from yeast. For example, yeast mannan formed by damaging yeast cell walls with an enzyme, chemical treatment, or the like may be separated and collected (extracted), and yeast mannan discharged by a yeast into a medium may be collected.

Yeast mannan is readily available as a commercially available product. Specific examples thereof include mannan from Saccharomyces cerevisiae M7504 (Sigma-Aldrich Japan). Alternatively, yeast mannan can be prepared from a yeast by using any known method as described in the following.

### (2) Method for Preparing Yeast Mannan

Any known method can be used for preparing yeast mannan. For example, yeast mannan can be obtained through a step of preparing cell walls from a yeast by extracting yeast extract from a yeast used in production of any of beer, sake, bread, and so on to afford residual yeast cell walls, and a step of preparing yeast mannan from the residual yeast cell walls. Yeast cell walls sold under the product name "Kobo Saibo Heki (Yeast Cell Walls in English)" by Asahi Group Foods, Ltd. and other similar commercially available products may be used as a raw material of yeast mannan.

An example of the step of preparing yeast mannan from yeast cell walls is a step including a step of obtaining a crude extract of yeast mannan from yeast cell walls (herein, called the "crude extraction step") and, as necessary, an additional subsequent step of purifying the crude extract of yeast mannan to obtain a purified product of yeast mannan (herein, called the "purification step"). In some cases, a step of preparing cell walls from a yeast is performed before the crude extraction step; however, such a step is unnecessary in the case that yeast cell walls produced in advance are used as a raw material. In the crude extraction step, for example, methods of hot water (including dilute alkalis) extraction (e.g., see JP 1989(S64)-3479 B and JP 1983(S58)-109423 A), autodigestion (e.g., see JP 1983(S58)-57153 B), and enzymatic digestion with a cell-wall-digesting enzyme (e.g., see JP 1983(S59)-40126 B) can be used. In the purification step, for example, protein removal treatment with hydrochloric acid or the like, alcohol precipitation treatment, protease treatment, and treatment with chromatography can be performed.

Herein, crude extracts of yeast mannan and purified products of yeast mannan are collectively called "yeast mannan preparations". The above-described commercially available yeast mannan products are also included in the category of yeast mannan preparations. Various contaminants present in yeast cell walls or generated through treatment for preparation of yeast mannan (e.g., glucan, protein, mannose) may be contained in addition to yeast-derived α-mannan and/or an α-mannan-protein complex in yeast mannan preparations. The yeast mannan content of a yeast mannan preparation is not limited to particular values, and may be any content such that the yeast mannan contains such a content of yeast α-mannan, described later, that the operative advantageous effects of the present invention are exerted when the yeast mannan preparation is used as yeast mannan. Which method is used in the crude extraction step, whether the purification step is performed, which treatment is used if the purification step is performed, and so on may be appropriately determined so that a yeast mannan preparation containing a desired content of yeast α-mannan can be obtained. Those skilled in the art could prepare yeast mannan containing such a content of yeast α-mannan that the operative advantageous effects of the present invention are exerted without problem in accordance with a common method and conditions. In other words, yeast mannan prepared by those skilled in the art in accordance with a common method and conditions contains such a content of yeast α-mannan that the operative advantageous effects of the present invention are exerted without problem.

The yeast to be used as a raw material in the above method for preparing yeast mannan is not limited to a particular yeast, and may be any yeast whose cell walls contain the above-described α-mannan and the above-described α-mannan-protein complex as constituent components. The yeast may be any yeast of Saccharomyces spp., Shizosaccharomyces spp., Pichia spp., Candida spp., Kluyveromyces spp., Williopsis spp., Debaryomyces spp., Galactomyces spp., Torulaspora spp., Rhodotorula spp., Yarrowia spp., Zygosaccharomyces spp., and so on, and is preferably Saccharomyces sp. Those skilled in the art could culture a yeast that does not cause any problem in using the yeast as a raw material for preparing yeast mannan that exerts the operative advantageous effects of the present invention in accordance with a common method and conditions. In other words, a yeast cultured by those skilled in the art in accordance with a common method and conditions can be used as a raw material for preparing yeast mannan that exerts the operative advantageous effects of the present invention without problem.

The above-described yeast to be used as a raw material of yeast mannan is preferably an edible yeast, and more preferably a yeast used as a bread yeast or a beer yeast. Specific examples thereof include Saccharomyces cerevisiae, Schizosaccharomyces pombe, Saccharomyces pastorianus, Saccharomyces paradoxus, Saccharomyces mikatae, Saccharomyces bayanus, Saccharomyces kudriavzevii, Kluyveromyces lactis, Candida utilis, Candida albicans, Candida tropicalis, Candida lypolitica, and Candida sake. The above-described yeast to be used as a raw material of yeast mannan is preferably Saccharomyces cerevisiae, Saccharomyces pastorianus, or Saccharomyces bayanus.

### (3) Method for Calculating Content Ratio of Yeast α-Mannan

The yeast mannan preparation obtained with the above preparation method may contain a yeast-derived α-mannan-protein complex (i.e., a protein moiety bonding to an α-mannan chain). Herein, yeast-derived α-mannan and the α-mannan chain part of a yeast-derived α-mannan-protein complex are collectively referred to as "yeast α-mannan" ("yeast α-mannan" can be said to be pure yeast mannan). The content ratio of yeast α-mannan in yeast mannan somewhat varies among methods for preparing yeast mannan, but is in a generally constant range, typically, 30 to 95%. The content ratio of yeast α-mannan is not limited to particular values, and may be any value such that the operative advantageous effects of the present invention are exerted in using a yeast mannan preparation as yeast mannan. Those skilled in the art could prepare yeast mannan with such a content ratio of yeast α-mannan that the operative advantageous effects of the present invention are exerted without problem in accordance with a common method and conditions. In other words, yeast mannan prepared by those skilled in the art in accordance with a common method and conditions has such a content ratio of yeast α-mannan that the operative advantageous effects of the present invention are exerted without problem.

The content ratio of yeast α-mannan in a yeast mannan preparation is calculated in the following manner. First, mannose in sample (A) of a yeast mannan preparation and mannose in sample (B) of a hydrolysis product of the yeast mannan preparation are quantified. Here, sample (B) is a specimen obtained by treating sample (A) with sulfuric acid to hydrolyze all the glycans in sample (A) into monosaccharides and then neutralizing the resultant. Mannose in each specimen can be quantified, for example, by using high-performance liquid chromatography (HPLC), and may be quantified by using a method performed in the general incorporated foundation Japan Food Research Laboratories. Subsequently, the amount of mannose in sample (A) is subtracted from the amount of mannose in sample (B), and the resulting value is multiplied by 0.9 to exclude the amount of water bonded through hydrolysis of yeast α-mannan, giving a calculated value of the content of yeast α-mannan in the yeast mannan preparation. Finally, the ratio of the content of yeast α-mannan to the weight of sample (A) is calculated, thus determining the ratio of yeast α-mannan in the yeast mannan preparation, that is, the content ratio of yeast α-mannan.

It is not needed to use a yeast mannan preparation with the content ratio of yeast α-mannan being 100% for the agents of the present invention, and any yeast mannan preparation with the content ratio of yeast α-mannan being in the above common range may be used.

The dosage form of the mucin generation promoting agent and fucosylation promoting agent of the present invention is not limited to a particular dosage form, and may be a powder or a solution (dispersion) using water or other solvent. The mucin generation promoting agent and fucosylation promoting agent of the present invention is typically blended in an intestinal-barrier-function-enhancing food product composition for use, and hence is preferably in a dosage form suitable for production of such food product compositions.

The mucin generation promoting agent and fucosylation promoting agent of the present invention, or yeast mannan contained as an active ingredient of them can promote generation of mucin and fucosylation in the intestinal tract of any of humans, mice, and other mammals through being ingested. The production of mucin and expression level of the Fut2 gene involved in fucosylation vary among test conditions such as feed conditions in a test with experimental animals, and it is difficult to rigidly specify the effect with a numerical range or the like. However, it is suitable that the mucin production after ingestion of the mucin generation promoting agent and the expression level of the Fut2 gene after ingestion of the fucosylation promoting agent be at least increased by a statistically significant difference as compared with those before ingestion under test conditions appropriately set to allow comparison. Alternatively, it is suitable that the mucin production after ingestion of the mucin generation promoting agent and the expression level of the Fut2 gene after ingestion of the fucosylation promoting agent be higher by a statistically significant difference than those for a control group (a group without ingestion of the mucin generation promoting agent and the fucosylation promoting agent), preferably those for a group with additional ingestion of another component such as "indigestible dextrin".

The mucin production and the expression level of the Fut2 gene can be measured by using any technique commonly used for analysis of them. The mucin production in the intestinal tract can be evaluated from, for example, the amount of mucin in a fecal specimen, and kits therefor (e.g., "Fecal Mucin Assay Kit" from COSMO BIO CO., LTD.) are commercially available. The Fut2 gene expression level can be measured, for example, in such a manner that a cDNA is prepared from a total RNA in ileum tissue and the Fut2 gene included in the cDNA is amplified through real-time PCR with appropriate primers.

### [Food Product Composition]

The food product composition for intestinal barrier function enhancement of the present invention contains the mucin generation promoting agent or fucosylation promoting agent of the present invention and an additional component compatible with the form of the food product composition. It should be noted that the scope of the food product composition of the present invention does not encompass natural materials and cooked or processed products thereof containing yeast mannan, which is the active ingredient of the mucin generation promoting agent or fucosylation promoting agent, in the form of a yeast with cell walls but produced not as a composition, such as fermented food products *per se* in which a yeast remains.

The form of the food product composition is not limited to a particular form, and the food product composition may be in any form of known food/beverage products in which the mucin generation promoting agent or fucosylation promoting agent of the present invention can be blended. Examples of the form of the food product composition include supplements (dietary supplements) in tablets, capsules, powders (granules), and other dosage forms; soft drinks (e.g., carbonated drinks, non-alcoholic drinks, juices, coffee drinks, tea drinks, mineral waters, sports drinks), milk drinks, soymilk drinks, fermented milks, lactic acid bacteria drinks, cocoas, alcoholic drinks (e.g., beer, low-malt beer, other brewed alcoholic drinks, liqueur, Japanese sake, amazake, wine, shochu), drinks prepared from instant powders, and other drinks; bread, cereal, confectionery (e.g., biscuits, cookies, chocolates), processed products (e.g., katsuobushi, shiokara, kusaya), salted vegetables (e.g., nukadzuke, kimchi, pickles), fermented food products (e.g., natto, cheese, yogurt), seasonings (e.g., soy sauce, miso, vinegar), food additives, and other food products.

The content of the mucin generation promoting agent or fucosylation promoting agent in the food product composition and the content of yeast mannan as the active ingredient are not limited to particular values, and each may be any value such that the mucin-generation-promoting effect or fucosylation-promoting effect of the present invention are exerted. The contents may be appropriately set in view of the form of and production method for the food product composition and, as necessary, the amount of the active ingredient per meal or day and so on. In using a yeast mannan preparation as the mucin generation promoting agent or fucosylation promoting agent of the present invention, the content of yeast α-mannan as an active ingredient can be controlled within such a range that the operative advantageous effects of the present invention are exerted, via the weight of the yeast mannan preparation and the ratio of yeast α-mannan contained in the yeast mannan preparation (i.e., the content ratio of yeast α-mannan).

In the case that the food product composition of the present invention is a supplement, for example, the content of the mucin generation promoting agent or fucosylation promoting agent can be such that the content of yeast α-mannan contained therein is typically 5% or more, preferably 10% or more, and typically 75% or less, to the total mass of the food product composition (supplement). In the case that the food product composition of the present invention is a beverage, the content of the mucin generation promoting agent or fucosylation promoting agent can be such that the content of yeast α-mannan contained therein is typically 0.04% or more, preferably 0.1% or more, and typically 7.5% or less, preferably 4% or less, to the total mass of the food product composition (beverage).

Raw materials or components of the food product composition of the present invention other than the mucin generation promoting agent or fucosylation promoting agent may be the same as those of the corresponding conventional food product compositions.

For example, the food product composition of the present invention in the form of a supplement may contain, in addition to the mucin generation promoting agent or fucosylation promoting agent, at least one selected from components contained in common or known supplements, such as excipients, sweeteners, coloring agents, flavors, and other components.

The food product composition of the present invention in the form of a beverage may contain, in addition to the mucin generation promoting agent or fucosylation promoting agent, at least one selected from components contained in common or known beverages, such as water (carbonated water), coffee extract, tea leaf extract, fruit juice, other extract of plant raw material, milk, soymilk, fermented milk, sweeteners, acidulants, coloring agents, and flavors.

The food product composition of the present invention as described can be produced in the same manner as the corresponding conventional food product compositions are produced, except that a step of adding as a component the mucin generation promoting agent or fucosylation promoting agent of the present invention is further included.

The food product composition may be produced, for example, as a food with health claims (i.e., a food with nutrient function claims, a food for specified health uses, or a food with functional claims), or a therapeutic diet (i.e., a diet for treatment, or a diet prepared on the basis of a menu made by a nutritionist or the like with reference to a dietary slip from a physician), a diet for dietary therapy, or a care diet.

The standard intake of the food product composition (the amount of the active ingredient) per meal or per day may be appropriately set, for example, according to the age, body weight, and sex of a subject whose intestinal barrier function is to be enhanced, and an index relating to the intestinal barrier function before ingestion for the subject, or on the basis of non-clinical or clinical test results or the like. The period to ingest the food product composition may be appropriately set, and it is preferred to ingest repeatedly on a daily basis for a long period of time. In one aspect of the present invention, the food product composition of the present invention is ingested so that the intake of yeast α-mannan contained therein as an active ingredient is preferably 0.1 g/day or more, more preferably 0.2 g/day or more, even more preferably 0.3 g/day or more, and, preferably 4 g/day or less, more preferably 2.5 g/day or less, even more preferably 1.5 g/day or less.

The use of the food product composition of the present invention, "enhancement of intestinal barrier function", may be displayed directly or indirectly. Examples of direct display include description printed on a tangible object such as a product itself, a package, a container, a label, and a tag, and examples of indirect display include publicity and advertisement in places or by means, for example, on a website, in a store, in an exhibition, with a billboard, with a bulletin board, in a newspaper, in a magazine, on TV, on radio, with a mail, or with an e-mail. In producing the food product composition of the present invention as a food with functional claims, for example, functions based on the operative advantageous effects of yeast mannan contained as a functional substance (promotion of mucin generation or promotion of fucosylation) may be displayed as "thicken the barrier of the intestine", "reinforce the barrier of the intestine", "support the health of the intestine", "make a strong body through the intestine", "achieve healthy constitution through the intestine", "enhance the barrier function of the intestine", "keep a healthy microbiome", "keep the microbiome healthy", "make the intestine resistant to risks", "strengthen the defense of the intestine", or the like.

### [Another Aspect of Present Invention]

The present invention provides, as one aspect, a method for promoting generation of mucin in the intestinal tract of a human or a non-human mammal (e.g., an experimental animal such as a mouse, or a pet), the method containing allowing the human or non-human mammal to ingest an effective amount of yeast mannan. The present invention provides, as one aspect, a method for promoting fucosylation in the intestinal tract of a human or a non-human mammal (e.g., an experimental animal such as a mouse, or a pet), the method containing allowing the human or non-human mammal to ingest an effective amount of yeast mannan. Those skilled in the art could divert the inventions of "mucin generation promoting agent" and "fucosylation promoting agent" described above to those inventions of "method for promoting mucin generation" and "method for promoting fucosylation", and the matters on the "mucin generation promoting agent" and "fucosylation promoting agent" described herein can be applied in the same manner.

The present invention provides, as one aspect, a method for enhancing the intestinal barrier function of a human or a non-human mammal (e.g., an experimental animal such as a mouse, or a pet), the method containing allowing the human or non-human mammal to ingest an effective amount of yeast mannan (or the mucin generation promoting agent and/or fucosylation promoting agent of the present invention). Those skilled in the art could divert the invention of "food product composition for intestinal barrier function enhancement" described above to the "method for enhancing intestinal barrier function", and the matters on the "food product composition for intestinal barrier function enhancement" described herein can be applied in the same manner.

The present invention provides, as one aspect, use of yeast mannan in production of a food product composition for enhancing the intestinal barrier function of a human or a non-human mammal (e.g., an experimental animal such as a mouse, or a pet). The present invention provides, as one aspect, use of yeast mannan as an active ingredient for promoting generation of mucin and/or promoting fucosylation in the intestinal tract of a human or a non-human mammal in production of the food product composition. Those skilled in the art could divert the inventions of "mucin generation promoting agent", "fucosylation promoting agent", and "food product composition for intestinal barrier function enhancement" described above to such "use of yeast mannan", and the matters on the "mucin generation promoting agent", "fucosylation promoting agent", and "food product composition for intestinal barrier function enhancement" described herein can be applied in the same manner.

### Examples

### (Method for Calculating Content Ratio of Yeast α-Mannan)

In Examples, mannose in sample (A) of yeast mannan preparation X obtained by a manner described later and mannose in sample (B) of a hydrolysis product of yeast mannan preparation X were quantified through outsourcing to the general incorporated foundation Japan Food Research Laboratories. On the basis of the quantification results, the content ratio of yeast α-mannan in yeast mannan preparation X was calculated by using the above-described calculation method.

The specific procedure of the quantification method for mannose in sample (A) and sample (B) by the general incorporated foundation Japan Food Research Laboratories was as follows. From each of sample (A) and sample (B), a portion of 0.6 g was taken, and each portion was added to sulfuric acid with a concentration of 72%, and the resultant was stirred at room temperature for 1 hour. The sulfuric acid concentration of each specimen solution was diluted to 4% by dilution, and the specimen solutions were then heated in an autoclave (121°C) for 1 hour. The specimen solutions were cooled to room temperature and then neutralized, and water was added to each specimen solution to adjust the volume to 200 mL. The specimen solutions after volume adjustment were each filtered through a filter paper, and the filtrates were each 50-fold diluted with water, and the diluted solutions were each filtrated through a membrane filter. The filtrates were analyzed by high-performance liquid chromatography under the following operation conditions to measure the mannose concentration of each filtrate, and mannose in sample (A) and sample (B) was quantified.
Apparatus: LC-20AD (Shimadzu Corporation)
Detector: fluorescence spectrophotometer RF-20Axs (Shimadzu Corporation)
Column: TSKgel SUGAR AXI, φ4.6 mm × 150 mm (Tosoh Corporation)
Column temperature: 60°C
Mobile phase: 0.5 mol/L borate buffer (pH 8.7)
Flow rate: 0.4 mL/min
Injection volume: 20 µL
Fluorescence excitation wavelength: 320 nm
Fluorescence measurement wavelength: 430 nm
Post-column:
reaction solution: 1 w/v% L-arginine solution;
flow rate of reaction solution: 0.7 mL/min;
reaction temperature: 150°C

### (Yeast Mannan Preparation X)

Ten kilograms of commercially available yeast cell walls (product name: "Kobo Saibo Heki (Yeast Cell Walls in English)", Asahi Group Foods, Ltd.) was suspended in distilled water, and sodium hydroxide was added to the suspension to adjust the pH to 11.0, and the suspension was then boiled at 90°C for 12 hours. The supernatant was collected through centrifugation, and hydrochloric acid was added thereto to adjust the pH to 5.5. A freeze-drying operation provided 2 kg of yeast mannan preparation X. By using the above-described method, the content ratio of yeast α-mannan was calculated to be 50%.

"Yeast mannan (2%)" and "Yeast mannan (5%)" in Examples 1 and 2 indicate that yeast mannan preparation X was mixed with a feed ("AIN93G", produced by Oriental Yeast Co., Ltd.) so that the mass of yeast mannan preparation X was 2% of the mass of the feed in the former case, and 5% of the mass of the feed in the latter case.

### (Cellulose and Indigestible Dextrin)

Cellulose ("CEOLUS UF-F702" produced by Asahi Kasei Chemicals Corporation) was used in Comparative Example 1. Indigestible dextrin ("Fibersol-2" produced by Matsutani Chemical Industry Co., Ltd.) was used in Comparative Example 2.

"Cellulose (5%)" and "indigestible dextrin (5%)" in Comparative Examples 1 and 2 indicate the weight or mass ratios of cellulose and indigestible dextrin, respectively, mixed in a feed ("AIN93G", Oriental Yeast Co., Ltd.) to the feed.

### (Experiment 1) Promotion of Mucin Generation

As described above, yeast mannan preparation X, cellulose, and indigestible dextrin were each mixed with a feed ("AIN93G", Oriental Yeast Co., Ltd.) to prepare test feeds. Four-week-old male ICR mice were grouped so that each group consisted of nine mice, and allowed to freely ingest each test feed for 6 weeks. Feces obtained before and after ingestion of the test feeds were freeze-dried and pulverized, and mucin was quantified by using a Fecal Mucin Assay Kit (FFA-MU-K01) produced by COSMO BIO CO., LTD. Amounts of mucin in feces 6 weeks after the ingestion were analyzed with Tukey-Kramer test to determine whether any significant difference was present among the group with administration of 5% cellulose (Comparative Example 1, a control group), the group with administration of 5% yeast mannan (Example 1), and the group with administration of 5% indigestible dextrin (Comparative Example 2).

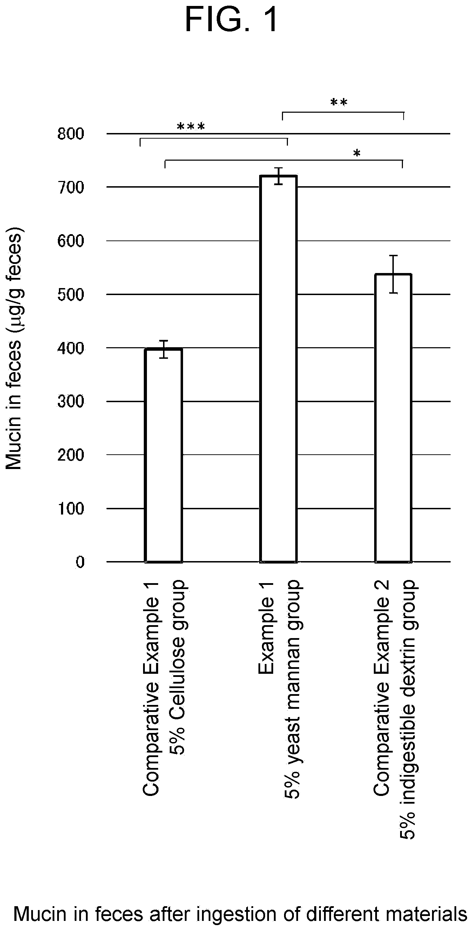

Table 1 and Figure 1 show the results. The results demonstrate that the amount of mucin in feces from the group provided with 5% yeast mannan (Example 1) was significantly higher than both that from the group with administration of 5% cellulose (Comparative Example 1) and that from the group with administration of 5% yeast mannan (Comparative Example 2), and hence that the mucin generation was promoted in the intestinal tract.

### [Table 1]

**Table 1. Amount of mucin per gram of feces before and after ingestion of different materials**

| | | Before ingestion (µg/g feces) | After 6 weeks ingestion (µg/g feces) |
|---|---|---|---|
| Example 1 | yeast mannan (2%) | 454±19 | 477±14 |
| Example 2 | yeast mannan (5%) | 516±28 | 720±15 |
| Comparative Example 1 | cellulose (5%) | 432±18 | 397±16 |
| Comparative Example 2 | indigestible dextrin (5%) | 462±13 | 537±35 |

### (Experiment 2) Promotion of Fucosylation

As described above, yeast mannan preparation X, cellulose, and indigestible dextrin were each mixed with a feed ("AIN93G", Oriental Yeast Co., Ltd.) to prepare test feeds. Four-week-old male ICR mice were grouped so that each group consisted of nine mice, and allowed to freely ingest each test feed for 6 weeks. After 6 weeks, each mouse was euthanized by cervical spine dislocation, and the ileum was cut out into a piece of 2.0 cm in length and placed in a tube containing 2.5 mL of RNA later solution, which was then refrigerated at 4°C for 24 hours and thereafter frozen at - 80°C.

The ileum tissue soaked in RNA later and frozen was finely cut, and 30 mg thereof was then weighed, and the total RNA was purified by using an "RNeasy Fibrous Tissue Mini Kit" produced by QIAGEN. Next, a cDNA was prepared from the total RNA by using "High Capacity cDNA Reverse Transcription Kits" produced by Applied Biosystems. PCR reaction was performed in accordance with the protocol by using the cDNA obtained, as a template, and "TaqMan Gene Expression Master Mix" and "TaqMan Gene Expression Assays" (primer for Fut2 gene: Mm00490152_s1, primer for GAPDH gene: Mm99999915_g1) produced by Applied Biosystems. For reaction and detection, a quantitative PCR apparatus ("Applied Biosystems 7500Fast Real-Time PCR System" produced by Thermo Fisher Scientific) was used.

Relative mRNA levels of the fucosyltransferase Fut2 gene were calculated with a comparative Ct method described in the instruction attached to the quantitative PCR apparatus. mRNA levels of the fucosyltransferase Fut2 gene in each group were normalized by measuring mRNA levels of a GAPDH (glyceraldehyde-3-phosphate dehydrogenase) gene as an internal standard. The normalized mRNA level (expression level) of the fucosyltransferase Fut2 gene for the group with administration of 5% cellulose (Comparative Example 1, a control group) was assumed as 1.0, and relative values thereto were determined for the group with administration of 5% yeast mannan (Example 1) and the group with administration of 5% indigestible dextrin (Comparative Example 2). Analysis was performed with Dunnett's test to determine whether any significant difference was present between expression levels (relative values) in Examples 1 and 2 and that in Comparative Example 1.

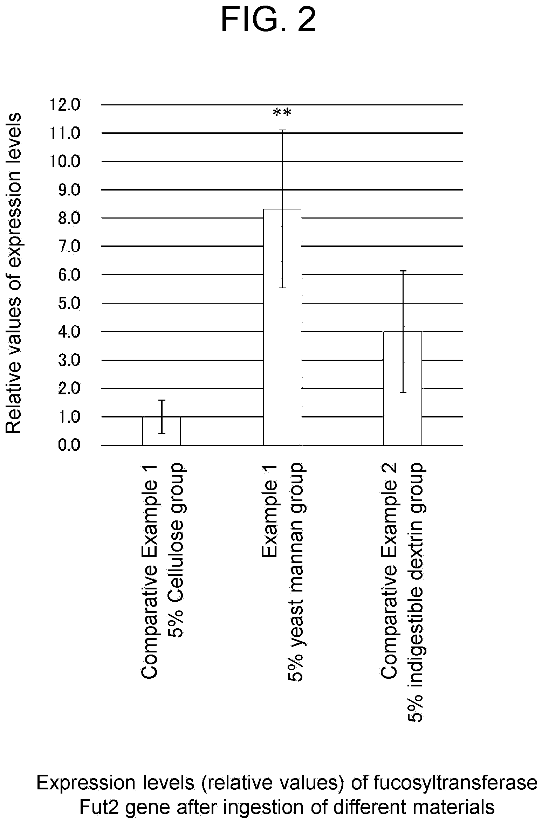

Figure 2 shows the results (the sign ** indicates the presence of a significant difference with a significance level of lower than 1% (P < 0.01)). The results demonstrate that the expression level of the fucosyltransferase Fut2 gene in ileum tissue from the group provided with 5% yeast mannan (Example 1) was significantly higher, and hence that fucosylation in the ileum was promoted.

## Claims

1. A mucin generation promoting agent comprising yeast mannan.

2. A fucosylation promoting agent comprising yeast mannan.

3. A food product composition for intestinal barrier function enhancement comprising the mucin generation promoting agent according to claim 1 or the fucosylation promoting agent according to claim 2.
